# EUROPEAN PATENT SPECIFICATION

(11) **EP 2 266 581 B1**
(45) Date of publication and mention of the grant of the patent: **23.09.2015**
(21) Application number: 08873478.5
(22) Date of filing: 18.03.2008
(51) Int. Cl.: A61K 47/26, A61K 31/7016, A61K 45/06, A61P 1/00

(54) **PHARMACEUTICAL COMPOSITION OF ANTIBIOTICS AND PREBIOTICS FOR PREVENTING AND TREATING DYSBIOSIS DURING ANTIBACTERIAL THERAPY**
PHARMAZEUTISCHE ZUSAMMENSETZUNG VON ANTIBIOTIKA UND PRÄBIOTIKA ZUR PRÄVENTION UND BEHANDLUNG VON DYSBIOSE WÄHREND EINER ANTIBAKTERIELLEN THERAPIE
COMPOSITION PHARMACEUTIQUE À BASE D'ANTIBIOTIQUES ET DE PRÉBIOTIQUES DESTINÉE AU TRAITEMENT DE DYSBIOSES DANS UN PROCESSUS DE THÉRAPIE ANTIBACTÉRIENNE

(43) Date of publication of application: 29.12.2010
(73) Proprietor: Dikovskiy, Aleksander Vladimirovich, Moscow 123182 (RU)
(72) Inventor: DOROZHKO, Oleg Valentinovich, Moscow 115446 (RU); RUDOI, Boris Anatolievich, Moscow 109518 (RU)
(74) Representative: Ackermann, Joachim
(86) International application number: PCT/RU2008/000152
(87) International publication number: WO 2009/116887

(56) References cited:
- EP-A1- 1 166 800
- RU-C2- 2 277 914
- RU-C2- 2 284 832
- US-A- 5 480 491
- ZOPPI GIUSEPPE ET AL: "Modulation of the intestinal ecosystem by probiotics and lactulose in children during treatment with ceftriaxone", CURRENT THERAPEUTIC RESEARCH, vol. 62, no. 5, May 2001 (2001-05), pages 418-435, XP002630603, ISSN: 0011-393X
- DASARATHY S: "ROLE OF GUT BACTERIA IN THE THERAPY OF HEPATIC ENCEPHALOPATHY WITH LACTULOSE AND ANTIBIOTICS", INDIAN JOURNAL OF GASTROENTEROLOGY, INDIAN SOCIETY OF GASTROENTEROLOGY, BOMBAY, IN, vol. 22, no. SUPPL. 02, 1 December 2003 (2003-12-01), pages S50-S53, XP009058694, ISSN: 0254-8860
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### Field of industry to which the invention relates

The group of inventions relates to medicine, namely to pharmaceutics and the development of compositions of pharmaceutical preparations containing antibiotics and prebiotics, for correction of the composition of the intestinal microflora during antibiotic therapy.

### Prior art

The use of broad-spectrum antibiotics for treatment of infections and other diseases is as a rule accompanied by gastrointestinal side-effects , which are mainly connected with the negative impact of antimicrobial preparations on the microflora of the intestine and other cavities. When taken orally, antibiotics eliminate not only pathogenic, but also indigenous microflora of the digestive tract, leading to disturbances of homeostasis and promote the development of dysbiosis and allergic reactions. Imbalance in the microbiocenosis of the intestine leads in many cases to disturbances in the immune system status, as well as to active multiplication of the unicellular fungi that colonize the mucosa of the large intestine.

It is known that maintaining of the normal microflora of the intestine in an active state is a necessary condition not only for digestion and the assimilation of nutrients, but also for preservation of a barrier preventing colonization of the intestine by pathogenic microorganisms. The intestinal microbiota also participates in metabolic detoxication of a number of toxic products, also it takes part in the assimilation of minerals and in the biosynthesis of some vitamins, and restricts the multiplication of pathogenic and opportunistic species of microorganisms living parasitically in the intestine.

The most favorable conditions for the activity of the microflora are arised in the intestine beginning from the distal portions of the small intestine, where the secretions of the stomach and proteases from the pancreas do not reach, as well as the components of bile, the bacteriostatic and bactericidal effects of which get weaker as the large intestine is approached. Against a background of dysbiosis, pathogens of intestinal infections or opportunistic microorganisms that have entered the body quickly colonize the mucosa of the small and large intestine, destroying epithelial cells and displaying pronounced antagonism with respect to the indigenous microflora. Inflammation develops, leading to decreased production of short-chain fatty acids, which are known to inhibit the growth of pathogenic microorganisms. This process occurs during antibiotic therapy with broad-spectrum preparations that are taken orally.

Numerous investigations have established that even partial loss of the normal intestinal microflora leads to serious consequences for the organism and requires special treatment.

Restoration of the intestinal microflora is often attained by prescribing various probiotic microorganisms, which are not always compatible with the representatives of the individual normal microflora and over a period of several days may eliminate them from the intestine. As for the development of undesirable effects when using these preparations, they are caused by the ability of probiotics to modulate immune inflammation. For example, it is known that in 10% of workers at factories producing bacterial preparations (probiotics) and immunobiological preparations, after several years of contact with bacteria there is development of allergic dermatitis.

A more physiological means of maintaining an active state of the normal intestinal microflora is ingestion of prebiotics. Prebiotics are indigestible ingredients of food, which promote improvement of health by selectively stimulating the growth and/or metabolic activity of one or more dominant species of bacteria living in the large intestine.

Prebiotics are not absorbed in the stomach, and pass through to the large intestine, and in the human body, owing to the absence of beta-glycosidases, they are not assimilated, but are used by the microflora of the large intestine as nutrient substrates. Prebiotics are able to selectively stimulate the growth and multiplication of lactobacilli and bifidobacteria, i.e. species that predominate in the composition of the normal human intestinal microflora.

At present, an ever increasing number of food supplements and functional foods, containing saccharides and oligosaccharides, in particular lactulose, as effective prebiotics, is appearing in the pharmaceutical marketplace, which reflects the good prospects of this direction for correcting disturbances of the microbiocenosis of the human intestine.

Lactulose performs prebiotic functions because it is barely cleaved by the enzymes in the upper regions of the GI tract and reaches the large intestine unchanged. There it undergoes a process of fermentation by bifidobacteria and serves as a growth factor for them. Fermentation takes place by anaerobic processes. Lactulose undergoes hydrolysis by bacterial enzymes with formation of organic acids, mainly lactic, acetic, butyric and propionic. As a result, the intestinal contents are acidified and the osmotic pressure in the large intestine increases.

Prescribing combination therapy with inclusion of lactulose is directed at eliminating dysbiosis, atrophic processes in the mucosa of the large intestine, and dystrophic changes in the epithelium with restoration of its functionality. However, taking antibiotics and lactulose at different times in at least half of cases cannot exclude damage to the intestinal microflora by the antibiotics. Most often, prebiotics are resorted only to after symptoms of dysbiosis have developed in the form of diarrhea and flatulence. As a result, by the moment when lactulose begins to be taken, after antibiotic therapy has been carried out, the useful microflora is substantially disrupted or is practically unviable.

Accordingly, it is actually urgent to provide selective advantages for the useful microflora over pathogenic or opportunistic bacterial species during therapy with antibiotics. Therefore there are attempts to provide protection of the indigenous intestinal microflora by simultaneous administration of an antibiotic and lactulose in the form of a single pharmaceutical composition.

There is a known pharmaceutical composition, the method of preparation thereof and method of use, comprising a medicinal product and saccharides (lactulose). The active ingredient and saccharides are prepared in a special pharmaceutical form coated with a polymeric material that is soluble in organic acids. In this composition the saccharides are fermented by enterobacteria with formation of organic acids, which activate the pharmaceutical preparation. The composition is characterized by highly specific delivery of the pharmaceutical agent into the distal portion of the large intestine (RU No. 2155605, 2000).

Drawbacks of said composition include the narrow range of use, limited spectrum of pharmaceutical agents that are only effective in the large intestine, absence of antibiotic activity and therefore impossibility of application in the treatment of diseases caused by or complicated by infectious agents, low specificity of the stimulating action on the main species of indigenous microflora, nonoptimal proportions by weight (lack of balance) of the medicinal product and lactulose, nonoptimal degree of dispersion of the saccharides, resulting in reduced level of fermentation and of the therapeutic-prophylactic effect from taking the composition, as well as insufficient absorption of calcium and bone mineralization, presence of side-effects on the blood coagulation system (mainly prolongation of the partial prothrombin time and decrease in fibrinogen level), high incidence of allergic reactions, and complexity of the process of preparation and application.

There is a known pharmaceutical composition, method of preparation and method of use thereof, containing the prebiotic lactulose and an antibiotic from the group: penicillin, cephalosporins, tetracyclines, lincosamides, macrolides (RU No. 2284832, publ. 10.10.2006, the prior art).

Drawbacks of this composition are the narrow range of use, production of compositions with nonoptimal composition with respect to the antibiotic and nonoptimal (in the indefinitely wide range stated in said publication) weight ratio of antibiotic and lactulose (i.e. their lack of balance), so that the antibacterial action of the product is reduced and the frequency of allergic reactions is increased, and the complexity of the process of preparation and application. A negative aspect is the indeterminate degree of purity, i.e. the presence, in lactulose from the majority of domestic manufacturers, of admixtures (up to 40%) of lactose, fructose and galactose, which stimulate the growth of pathogenic and conditionally pathogenic bacteria of the intestinal group. Moreover, excessive laxative action of the composition owing to the unbalanced content of lactulose with said antibiotics, reduces the transit time of the intestinal contents and accordingly decreases the absorption and assimilation of nutrients, and therefore some external symptoms of dysbacteriosis may develop, diarrhea for example.

Shortcomings in efficacy of the composition are also due to the composition of the antibiotics recommended for the composition, since they (penicillin and cephalosporins) are more often used in injected form, when they act systemically and do not cause appreciable damage to the intestinal microflora. However, these antibiotics (tetracyclines and penicillin) have been known for more than fifty years and have already lost their clinical importance to a great extent in connection with widespread bacterial resistance to penicillin (production of the enzyme beta-lactamase) and to tetracyclines (R plasmids with the Tc gene), but they are causing allergic reactions increasingly frequent.

### Disclosure of the essence of the invention

The technical object of the group of inventions, bound by a common inventive concept, is the creation of an effective pharmaceutical composition and a method of production thereof for preventing enteral dysbiosis while extending the arsenal of pharmaceutical compositions.

The technical result that enables this object to be achieved comprises substantial expansion of the range of application of compositions of lactulose and antibiotics, by the inclusion in the composition of more effective antibacterial preparations for oral administration (fluoroquinolones, ansamycins etc.) and the elimination of side-effects. In addition, effective utilization of the prebiotic component of the composition in the intestine is provided, by a high degree of homogeneity (purification) of the lactulose, which precludes growth of pathogenic and opportunistic bacteria, as well as the use of optimal proportions of antibiotic and lactulose and an optimal degree of dispersion of the particles of the composition.

Use of this composition not only creates conditions for preservation and growth of the indigenous microflora during antibiotic therapy, but also effectively improves the composition of the blood, the state of the cardiovascular system, creates selective advantages for growth of useful species of microorganisms and inhibition of the growth of opportunistic and pathogenic bacterial species in the intestine.

The essence of the invention with respect to the pharmaceutical composition for oral application is that, according to a first embodiment, it comprises an antibiotic according to claim 1 and lactulose, the antibiotic having a particle size from 20 to 160 µm, and the lactulose having a particle size up to 0.3 mm and purity of at least 97%, moreover the weight ratio of antibiotic to lactulose is from 1:0.1 to 1:100.

The pharmaceutical composition of the first embodiment includes an antibiotic selected from the group comprising beta-lactams with inhibitors of bacterial beta-lactamases, fluoroquinolones, azalides, amphenicols, glycopeptides, ansamycins, nitrofurans, derivatives of phosphonic acid. Preferably the composition of the first embodiment additionally includes excipients selected from the group comprising fillers, taste correctants, flavorings, and odoriferous substances, used in pharmaceutically acceptable amounts; the composition is produced in a pharmaceutical form suitable for oral administration, selected from the group comprising capsules, tablets, powders, pills, sugar-coated pills, granules, sachets, gels, pastes, syrups, emulsions, suspensions and solutions; the pharmaceutical composition is taken orally 2-3 times a day.

The essence of the invention with respect to the pharmaceutical composition for oral application is that, according to a second embodiment, it comprises a sulfanilamide preparation and lactulose, the sulfanilamide preparation having particle size from 40 to 150 µm, and the lactulose having particle size up to 0.3 mm and purity of at least 97%, the weight ratio of sulfanilamide preparation to lactulose being 1:12.

Preferably it includes sulfadimezine/sulfazine as sulfanilamide preparation and additionally includes excipients selected from the group comprising fillers, taste correctants, flavorings, and odoriferous substances, used in pharmaceutically acceptable amounts, and prepared in a pharmaceutical form suitable for oral administration, selected from the group comprising capsules, tablets, powders, pills, sugar-coated pills, granules, sachets, gels, pastes, syrups, emulsions, suspensions and solutions; the pharmaceutical composition is administered orally 2-3 times a day.

According to the first embodiment the method of production of a pharmaceutical composition, comprising an antibiotic according to claim 8 and lactulose, the antibiotic having particle size from 20 to 160 µm, and the lactulose having particle size up to 0.3 mm and purity of at least 97%, the ratio of antibiotic to lactulose being from 1:0.1 to 1:100, is carried out by mixing the antibiotic and the lactulose, used in the form of powder; moreover the powdered antibiotic has particle size from 20 to 160 µm, and the powdered lactulose has particle size up to 0.3 mm and purity of at least 97%, at a weight ratio of antibiotic to lactulose from 1:0.1 to 1:100. Preferably the method additionally includes mixing with excipients selected from the group comprising fillers, taste correctants, flavorings, and odoriferous substances.

According to the second embodiment the method of production of a pharmaceutical composition, comprising a sulfanilamide preparation and lactulose, the sulfanilamide preparation being included with particle size from 40 to 150 µm, and the lactulose with particle size up to 0.3 mm and purity of at least 97%, the ratio of sulfanilamide preparation to lactulose being 1:12, is carried out by mixing the sulfanilamide preparation and the lactulose, used in the form of powder; moreover the powdered sulfanilamide preparation has particle size from 40 to 150 µm, and the powdered lactulose has particle size up to 0.3 mm and purity of at least 97%, with weight ratio of sulfanilamide preparation to lactulose of 1:12. Preferably the method additionally includes mixing with excipients selected from the group comprising fillers, taste correctants, flavorings, and odoriferous substances.

It is borne in mind that the human body is a multi-organ system, the elements of which are specialized for performing various functions. Interaction within the organism is achieved by complex neurohumoral regulation and correlating mechanisms involving metabolic and other factors. The many separate mechanisms regulating intracellular and intercellular interactions exert opposing effects, balancing one other. This maintains constancy of the internal environment in the organism and allows the system as a whole to maintain relative dynamic equilibrium, reacting to changes in the surroundings and shifts that arise in the course of activity. Disturbance of physiological balance, including that connected with disturbance of equilibrium in the microecology, may be manifested in the form of diseases of various organs. The proposed composition aims to exclude or effectively reduce any deviations from physiological balance in the state of the intestinal microflora under the influence of destructive factors in the form of antibiotics.

The saccharolytic intestinal microflora (lactobacilli and bifidobacteria) ferments lactulose by means of glycosidases, leading to an increase in the production of enzymes and an increase in the level of saccharolytic activity, with the lactulose exerting a dose-dependent bifidogenic effect.

Since the prebiotic - lactulose in the form specified according to the present invention - is present in the proposed composition together with antibiotic in the necessary weight ratio, while the antibiotic eradicates the pathogenic microorganisms, the intrinsic microflora of the large intestine is not destroyed, but synchronously with the arrival of the disaccharide it hydrolyzes (ferments) the latter with formation of an effective amount of organic acids (lactic, butyric, acetic and propionic), which acidify the contents of the large intestine. The osmotic pressure in the large intestine increases to 6.6-8.0 atm and the pH falls below 5.0, reliably maintaining the normal selective permeability of the biological membranes of the intestinal mucosa, retention of ammonium ions, and removal of ammonia from the blood into the intestine, thereby creating, in the lumen of the large intestine, entirely unfavorable conditions for the development of pathogenic bacteria, e.g. salmonella.

The acid products and other metabolites that have formed suppress the development of proteolytic microflora. As a result, there is a decrease in the amounts of pathogenic bacteria and toxic metabolites (ammonia, skatole, indole etc.) in the lumen of the intestine.

Against a background of effective maintenance of homeostasis, there is nothing to hamper adequate multiplication and stimulation of growth of the natural useful intestinal microflora that is to be preserved. With increase in acidity of the environment, the acid reacts with the amino groups of the protein and, by capturing OH ions, promotes the development of electropositive protein, which suppresses inflammatory processes that might develop in the intestine owing to external causes or as a complication of the underlying disease.

The process for preparation of the proposed composition envisages preparing specified amounts of the powdered antibiotic and powdered lactulose with purity of at least 97% guaranteed by the supplier, predrying to 2-3% moisture and mixing in the proportions specified by the present invention. Then anticaking additives, flavorings, and taste correctants are incorporated in the mixture, and static electric charges are removed.

Next, packaging is carried out in accordance with the dosage and pharmaceutical form.

Compositions were prepared with the following combinations of ingredients.

Lactulose with one of the amphenicols, with the oligosaccharide (hereinafter: lactulose) in the form of powder with particle size of 0.1-0.3 mm, and the antibiotic in the form of powder with particle size of 50-150 µm, the antibiotic and the lactulose being used in the weight ratio of 1:0.2.

Lactulose with one of the fluoroquinolones, with the oligosaccharide in the form of powder with particle size of 0.1-0.3 mm, and the antibiotic in the form of powder with particle size of 20-90 µm, the antibiotic and the lactulose being used in the weight ratio of 1:3.

Lactulose with one of the glycopeptides, with the oligosaccharide in the form of powder with particle size of 0.1-0.3 mm, and the antibiotic in the form of powder with particle size of 30-100 µm, the antibiotic and the lactulose being used in the weight ratio of 1:40.

Lactulose with one of the ansamycins, with the oligosaccharide in the form of powder with particle size of 0.1-0.3 mm, and the antibiotic in the form of powder with particle size of 20-110 µm, the antibiotic and the lactulose being used in the weight ratio of 1:60.

Lactulose with one of the derivatives of phosphonic acid (fosfomycin), with the oligosaccharide in the form of powder with particle size of 0.1-0.3 mm, and the antibiotic in the form of powder with particle size of 20-90 µm, the antibiotic and the lactulose being used in the weight ratio of 1:95.

Lactulose with one of the nitrofurans, with the lactulose in the form of powder with particle size of 0.1-0.3 mm, and the antibiotic in the form of powder with particle size of 60-160 µm, the antibiotic and the lactulose being used in the weight ratio of 1:5.5.

Lactulose with one of the sulfanilamide preparations (sulfadimezine), with the lactulose in the form of powder with particle size of 0.2-0.3 mm, and the sulfanilamide preparation in the form of powder with particle size of 40-150 µm, the sulfanilamide and the lactulose being used in the weight ratio of 1:12.

The composition was prepared in pharmaceutical forms suitable for oral use, in particular in the form of capsules, tablets, powders, pills, sugar-coated pills, granules, sachets, gel, paste, syrup, emulsion, suspension, solution. The compositions included pharmaceutically acceptable amounts of excipients for improving organoleptic properties, in particular: fillers, taste correctants, flavorings, and odoriferous substances.

For investigation of the therapeutic action of the compositions obtained and confirmation of their suitability as therapeutic and therapeutic-prophylactic agents, their effects on the condition of patients with various infectious diseases were investigated. This included assessment of the effects of the preparations on the general condition of the patients, physical activity, status of the cardiovascular system, neurologic status etc.

In the test group, 84 patients in the age range from 18 to 65 years were monitored: 32 men and 52 women. The diagnosis was made during outpatient assessment based on medical examination, results of laboratory and biochemical tests, ECG data, echocardiography etc.

28 patients had diagnosis of the gastritis, duodenitis, or gastric ulcer; 34 patients had diagnosis of angina and ARD; and 25 women had gynecological diagnoses. Many of these patients had had symptoms of gastrointestinal disturbances for a long time (colitis, enterocolitis, IBS etc.). In previous treatment, the use of antibiotics from the group comprising penicillins, cephalosporins, tetracyclines, lincosamides, and macrolides had proved ineffective, and as a rule caused allergic reactions.

Before they started intake of antibiotics in combination with lactulose according to the proposed composition, all patients had a general blood examination and biochemical analysis in samples of the blood serum were investigated: AST- and ALT-activity, concentration of creatinine, glucose, calcium, total bilirubin, protein, serum iron, TIBC, sodium, potassium, cholesterol, uric acid, urea, albumin; activity of alkaline phosphatase, GGT, triglycerides, β-lipoproteins, and in addition urine analysis, microbiologic analysis of the intestinal contents and microbiological investigation of the feces were carried out.

The patients took the preparation 2-3 times a day, during or after a meal in amounts specified in the label for preparation and the standard treatment regimens for specified infectious diseases. On average, antibiotics with lactulose according to each of these embodiments were taken by patients in test subgroups of 5-8 patients. Monitoring tests were carried out for 2 months, every 8-10 days.

Moreover, the first control group - 48 patients of similar age and physical condition, with the same diseases received antibiotics plus placebo (instead of lactulose), and the second control group - 44 patients of similar age and physical condition, with the same diseases - received antibiotics with lactulose separately, within an interval of 2-2.5 hours.

For the patients in both groups, during the first days of administration of the compositions their status was assessed as unsatisfactory, observing increase in body temperature, weakness, depression, flatulence, constipation or diarrhea (the latter arose, as a rule, after previous courses of treatment with antibiotics). Improvement in general ystatus was marked in the majority of patients in the test group after administration of the compositions during 5-8 days. On the 6-9 days after the start of treatment, the state of the patients in the control group had also improved with respect to the base disease, but 76% of patients in the first group and 52% of patients in the second group had clear symptoms of negative effects of the antibiotics on the intestinal microecology (dysbiosis), manifested as discomfort, slight pains along the large intestine, flatulence and diarrhea. In some patients the dysbiosis led to reduced appetite and sleep disturbance.

By the end of the course of treatment with the preparations, there was overall improvement of all patients status in the test group. There was a dramatic improvement in the status of 43 patients, and for the other patients in this group the symptoms of the main disease had practically disappeared. In 22 patients with various symptoms of atherosclerosis, headaches decreased in 12 cases, dizziness in 9, tinnitus in 8, cardiac pains in 14, and arterial pressure had normalized in 16 cases.

For the gynecological patients in the test group, the efficacy of the treatment was assessed before the start and after completion of treatment, using: biopsy of cervical mucosa, cytologic and microbiological investigation. After the first four weeks of intaking the preparation, appearance of the first sites of marginal epithelization of erosions was noted, and lactobacilli and bifidobacteria appeared in the microbiocenoses of the vagina, discharges decreased considerably and painful sensations had disappeared completely. The data from morphological investigation after stopping of the administration of the preparation indicated almost complete replacement of columnar epithelium with squamous epithelium. Smears indicated a decrease in signs of inflammation.

In nearly all the patients in the test group, the functional state of the gastrointestinal tract had normalized, and the amount of muscle fibers, fat, fatty acids, and undigested cellulose in the stool specimen was in the normal range. Analysis of the dynamics of a number of biochemical indices showed that there is a significant decrease in bilirubin, β-lipoproteins, triglycerides, ALT, AST. The structural-functional changes in the plasma proteins lead to enhancement of albumin binding capacity, and increase in the activity of antibodies and proteins of the complement system. The results of biochemical investigation and cell counts of the peripheral blood also indicate positive dynamics of the process.

At the same time there is an increase in the amount of urea synthesized, indicating improvement of the processes of reamination and transamination of amino acids in the liver, i.e. normalization of metabolic detoxication processes.

Nearly all patients in the test group noticed improvement in quality of life, increase in physical activity, improvement of mood and sleep, normalization of appetite and of the function of the intestines. No adverse side-effects from taking the compositions with antibiotics appeared during treatment or follow-up.

In the first control group, for patients who received the antibiotic plus placebo, despite the decrease in severity of the symptoms of the main disease owing to the influence of the antibiotic, in 87% of cases the indices of intraintestinal homeostasis did not show a clear tendency toward improvement. In the second control group, for patients who received the antibiotic and lactulose separately, the indices of intraintestinal homeostasis were only similar to the indices of the test group in 53% of cases.

Observation of the status of the majority of patients in the test group, who received the proposed preparation, continued over the next 18 months and confirmed the decrease in incidence of acute respiratory viral diseases, increase in performance capacity, normalization of sleep, decrease in frequency of relapses of the base disease and steady improvement in quality of life, especially in evacuation activity of the bowel.

When compared with the average age indices of morbidity, for patients in the test group it was found there was reduced incidence of oncologic diseases, cardiovascular diseases, diseases of the musculoskeletal system, as well as shorter recovery times from small injuries of the ligaments, bones and joints.

It can justifiably be concluded from the foregoing that the proposed composition is an effective and safe product for treatment of diseases caused by infectious agents, moreover the composition does not have a harmful effect on the microecology of the intestine, having a stimulant effect on lactobacilli and bifidobacteria and inhibiting the growth and multiplication of exogenous microflora and thus exerting a prophylactic action.

Thus, an effective pharmaceutical composition and method of preventing enteral dysbiosis have been created, and the arsenal of pharmaceutical compositions and methods of prevention of enteral dysbiosis has been extended.

Moreover, the range of application of compositions of lactulose and antibiotics has been extended by including more effective antibacterial preparations for oral administration (fluoroquinolones, ansamycins etc.) in the compositions, and eliminating side-effects. Furthermore, effective utilization of the prebiotic component of the composition in the intestine has been provided by using optimal proportions of antibiotic and lactulose and an optimal degree of dispersion of the particles of the composition. Moreover, the pharmaceutical composition has a stimulant action on the immune system, promotes reduction of inflammation, activates mineral metabolism and synthesis of vitamins, and normalizes the activity of the bowel, i.e. provides a prophylactic action.

### Suitability for industrial application

The present invention is implemented using universal equipment, which is widely used in industry.

## Claims

1. A pharmaceutical composition for preventing enteral dysbiosis during antibiotic therapy, **characterized in that** it comprises an antibiotic selected from the group comprising beta-lactams with inhibitors of bacterial beta-lactamases, fluoro-quinolones, azalides, amphenicols, glycopeptides, ansamycins, nitrofurans and derivatives of phosphonic acid and lactulose, with the antibiotic having particle size from 20 to 160 µm, and the lactulose having particle size of up to 0.3 mm and purity of at least 97%, the weight ratio of antibiotic to lactulose being from 1:0.1 to 1:100.

2. The pharmaceutical composition as claimed in claim 1, **characterized in that** it additionally comprises excipients selected from the group comprising fillers, taste correctants, flavorings, and odoriferous substances, used in pharmaceutically acceptable amounts.

3. The pharmaceutical composition as claimed in claim 1, **characterized in that** it is produced in a pharmaceutical form suitable for oral administration, selected from the group comprising capsules, tablets, powders, pills, sugar-coated pills, granules, sachets, gels, pastes, syrups, emulsions, suspensions and solutions, the pharmaceutical composition being administered orally 2-3 times a day.

4. A pharmaceutical composition for preventing enteral dysbiosis during antibiotic therapy, **characterized in that** it comprises a sulfanilamide preparation and lactulose, with the sulfanilamide preparation having particle size from 40 to 150 µm, and the lactulose having particle size up to 0.3 mm and purity of at least 97%, the weight ratio of sulfanilamide preparation to lactulose being 1:12.

5. The pharmaceutical composition as claimed in claim 4, **characterized in that** it comprises sulfadimezine as sulfanilamide preparation.

6. The pharmaceutical composition as claimed in either of claims 4 or 5, **characterized in that** it additionally comprises excipients selected from the group comprising fillers, taste correctants, flavorings, and odoriferous substances, used in pharmaceutically acceptable amounts.

7. The pharmaceutical composition as claimed in either of claims 4 or 5, **characterized in that** it is produced in a pharmaceutical form suitable for oral administration, selected from the group comprising capsules, tablets, powders, pills, sugar-coated pills, granules, sachets, gels, pastes, syrups, emulsions, suspensions and solutions, the pharmaceutical composition being administered orally 2-3 times a day.

8. A method of production of the pharmaceutical composition **characterized in** claim 1, by mixing an antibiotic selected from the group comprising beta-lactams with inhibitors of bacterial beta-lactamases, fluoro-quinolones, azalides, amphenicols, glycopeptides, ansamycins, nitrofurans and derivatives of phosphonic acid and lactulose, both used in the form of powder; moreover, the powdered antibiotic has particle size from 20 to 160 µm, and the lactulose has particle size up to 0.3 mm and purity of at least 97%, at a weight ratio of antibiotic to lactulose from 1:0.1 to 1:100.

9. The method of production of a pharmaceutical composition as claimed in claim 8, additionally comprising mixing with excipients selected from the group comprising fillers, taste correctants, flavorings, and odoriferous substances.

10. A method of production of the pharmaceutical composition **characterized in** claim 4, by mixing a sulfanilamide preparation and lactulose, both in the form of powder; moreover, the powdered sulfanilamide preparation has particle size from 40 to 150 µm, and the lactulose has particle size up to 0.3 mm and purity of at least 97%, with weight ratio of sulfanilamide preparation to lactulose of 1:12.

11. The method of production of a pharmaceutical composition as claimed in claim 10, additionally comprising mixing with excipients selected from the group comprising fillers, taste correctants, flavorings, and odoriferous substances.

## Patentansprüche

1. Pharmazeutische Zusammensetzung zum Verhindern von Enterodysbiose während der Antibiotikatherapie, **dadurch gekennzeichnet, dass** es ein Antibiotikum aus der Gruppe umfassend beta-Lactame mit Hemmern von bakteriellen beta-Lactamasen, Fluorchinolone, Azalide, Amphenicole, Glycopeptide, Ansamycine, Nitrofurane und Derivate der Phosphonsäure und Lactulose umfasst, wobei das Antibiotikum eine Teilchengröße von 20 bis 160 µm aufweist und die Lactulose eine Teilchengröße von bis zu 0,3 mm und eine Reinheit von mindestens 97% aufweist, wobei das Gewichtsverhältnis von Antibiotikum zu Lactulose 1:0,1 bis 1:100 beträgt.

2. Pharmazeutische Zusammensetzung nach Anspruch 1, **dadurch gekennzeichnet, dass** sie zusätzlich Grundstoffe, ausgewählt aus der Gruppe bestehend aus Füllstoffen, Geschmackskorrigenzien, Geschmacksstoffen und Duftstoffen, die in pharmazeutisch unbedenklichen Mengen verwendet werden, umfasst.

3. Pharmazeutische Zusammensetzung nach Anspruch 1, **dadurch gekennzeichnet, dass** sie in einer Darreichungsform hergestellt wird, die sich für die orale Verabreichung eignet, ausgewählt aus der Gruppe umfassend Kapseln, Tabletten, Pulver, Pillen, Dragees, Granulate, Pulverbriefchen, Gele, Pasten, Sirupe, Emulsionen, Suspensionen und Lösungen, wobei die pharmazeutische Zusammensetzung 2-3 mal pro Tag oral verabreicht wird.

4. Pharmazeutische Zusammensetzung zur Verhinderung von Enterodysbiose während der Antibiotikatherapie, **dadurch gekennzeichnet, dass** sie ein Sulfanilamidpräparat und Lactulose umfasst, wobei das Sulfanilamidpräparat eine Teilchengröße von 40 bis 150 µm aufweist und die Lactulose eine Teilchengröße von bis zu 0,3 mm und eine Reinheit von mindestens 97% aufweist, wobei das Gewichtsverhältnis des Sulfanilamidpräparats zu der Lactulose 1:12 beträgt.

5. Pharmazeutische Zusammensetzung nach Anspruch 4, **dadurch gekennzeichnet, dass** sie Sulfadimezin als Sulfanilamidpräparat umfasst.

6. Pharmazeutische Zusammensetzung nach einem der Ansprüche 4 oder 5, **dadurch gekennzeichnet, dass** sie zusätzlich Grundstoffe, ausgewählt aus der Gruppe umfassend Füllstoffe, Geschmackskorrigenzien, Geschmacksstoffe und Duftstoffe, die in pharmazeutisch unbedenklichen Mengen eingesetzt werden, umfasst.

7. Pharmazeutische Zusammensetzung nach einem der Ansprüche 4 oder 5, **dadurch gekennzeichnet, dass** sie in einer Darreichungsform hergestellt wird, die sich für die orale Verabreichung eignet, ausgewählt aus der Gruppe umfassend Kapseln, Tabletten, Pulver, Pillen, Dragees, Granulate, Pulverbriefchen, Gele, Pasten, Sirupe, Emulsionen, Suspensionen und Lösungen, wobei die pharmazeutische Zusammensetzung 2-3 mal pro Tag oral verabreicht wird.

8. Verfahren zur Herstellung der pharmazeutischen Zusammensetzung nach Anspruch 1 durch Mischen eines Antibiotikums, ausgewählt aus der Gruppe umfassend beta-Lactame mit Hemmern von bakteriellen beta-Lactamasen, Fluorochinolonen, Azaliden, Amphenicolen, Glycopeptiden, Ansamycinen, Nitrofuranen und Derivaten von Phosphonsäure und Lactulose, die beide in Form eines Pulvers verwendet werden und wobei weiterhin das pulverförmige Antibiotikum eine Teilchengröße von 20 bis 160 µm und die Lactulose eine Teilchengröße von bis zu 0,3 mm und eine Reinheit von mindestens 97% aufweist, im Gewichtsverhältnis von Antibiotikum zu Lactulose von 1:0,1 bis 1:100.

9. Verfahren zur Herstellung einer pharmazeutischen Zusammensetzung nach Anspruch 8, das zusätzlich das Mischen mit Grundstoffen, ausgewählt aus der Gruppe umfassend Füllstoffe, Geschmackskorrigenzien, Geschmacksstoffe und Duftstoffe, umfasst.

10. Verfahren zur Herstellung der pharmazeutischen Zusammensetzung nach Anspruch 4 durch Mischen eines Sulfanilamidpräparats und von Lactulose, beide in Pulverform, wobei weiterhin das pulverförmige Sulfanilamidpräparat eine Teilchengröße von 40 bis 150 µm und die Lactulose eine Teilchengröße von bis zu 0,3 mm und eine Reinheit von mindestens 97% aufweist, wobei das Gewichtsverhältnis von Sulfanilamidpräparat zu Lactulose 1:12 beträgt.

11. Verfahren zur Herstellung einer pharmazeutischen Zusammensetzung nach Anspruch 10, das zusätzlich das Mischen mit Grundstoffen, ausgewählt aus der Gruppe umfassend Füllstoffe, Geschmackskorrigenzien, Geschmacksstoffe und Duftstoffe, umfasst.

## Revendications

1. Composition pharmaceutique destinée à prévenir une dysbiose entérale durant une antibiothérapie, **caractérisée en ce qu'**elle comprend un antibiotique sélectionné parmi le groupe comprenant des bêta-lactames avec des inhibiteurs de bêta-lactamases bactériennes, des fluoroquinolones, des azalides, des amphénicols, des glycopeptides, des ansamycines, des nitrofuranes et des dérivés d'acide phosphonique et du lactulose, l'antibiotique présentant une taille de particules comprise entre 20 et 160 µm, et le lactulose présentant une taille de particules jusqu'à 0,3 mm et une pureté d'au moins 97 %, le rapport massique d'antibiotique sur lactulose étant compris entre 1:0,1 et 1:100.

2. Composition pharmaceutique selon la revendication 1, **caractérisée en ce qu'**elle comprend en outre des excipients sélectionnés parmi le groupe comprenant des agents de charge, des correcteurs de goût, des parfums, et des substances odoriférantes, utilisés selon des quantités pharmaceutiquement acceptables.

3. Composition pharmaceutique selon la revendication 1, **caractérisée en ce qu'**elle est produite selon une forme pharmaceutique adaptée à une administration orale, sélectionnée parmi le groupe comprenant des capsules, des comprimés, des poudres, des pilules, des pilules enrobées, des granules, des sachets, des gels, des pâtes, des sirops, des émulsions, des suspensions et des solutions, la composition pharmaceutique étant administrée oralement 2 à 3 fois par jour.

4. Composition pharmaceutique destinée à prévenir une dysbiose entérale durant une antibiothérapie, **caractérisée en ce qu'**elle comprend une préparation de sulfanilamide et du lactulose, la préparation de sulfanilamide présentant une taille de particules comprise entre 40 et 150 µm, et le lactulose présentant une taille de particules jusqu'à 0,3 mm et une pureté d'au moins 97 %, le rapport massique de préparation de sulfanilamide sur lactulose étant de 1:12.

5. Composition pharmaceutique selon la revendication 4, **caractérisée en ce qu'**elle comprend de la sulfadimézine en tant que préparation de sulfanilamide.

6. Composition pharmaceutique selon l'une ou l'autre des revendications 4 ou 5, **caractérisée en ce qu'**elle comprend en outre des excipients sélectionnés parmi le groupe comprenant des agents de charge, des correcteurs de goût, des parfums, et des substance odoriférantes, utilisés selon des quantités pharmaceutiquement acceptables.

7. Composition pharmaceutique selon l'une ou l'autre des revendications 4 ou 5, **caractérisée en ce qu'**elle est produite selon une forme pharmaceutique adaptée à une administration orale, sélectionnée parmi le groupe comprenant des capsules, des comprimés, des poudres, des pilules, des pilules enrobées, des granules, des sachets, des gels, des pâtes, des sirops, des émulsions, des suspensions et des solutions, la composition pharmaceutique étant administrée oralement 2 à 3 fois par jour.

8. Procédé de production de la composition pharmaceutique caractérisée selon la revendication 1, par mélange d'un antibiotique sélectionné parmi le groupe comprenant des bêta-lactames avec des inhibiteurs de bêta-lactamases bactériennes, des fluoroquinolones, des azalides, des amphénicols, des glycopeptides, des ansamycines, des nitrofuranes et des dérivés d'acide phosphonique et du lactulose, tous deux utilisés sous la forme de poudre ; de plus, l'antibiotique en poudre présente une taille de particules comprise entre 20 et 160 µm, et le lactulose présente une taille de particules jusqu'à 0,3 mm et une pureté d'au moins 97 %, à un rapport massique d'antibiotique sur lactulose compris entre 1:0,1 et 1:100.

9. Procédé de production d'une composition pharmaceutique selon la revendication 8, comprenant en outre un mélange avec des excipients sélectionnés parmi le groupe comprenant des agents de charge, des correcteurs de goût, des parfums, et des substances odoriférantes.

10. Procédé de production de la composition pharmaceutique caractérisée selon la revendication 4, par mélange d'une préparation de sulfanilamide et du lactulose, tous deux sous forme de poudre ; de plus, la préparation de sulfanilamide en poudre présente une taille de particules comprise entre 40 et 150 µm, et le lactulose présente une taille de particules jusqu'à 0,3 mm et une pureté d'au moins 97 %, le rapport massique de préparation de sulfanilamide sur lactulose étant de 1:12.

11. Procédé de production d'une composition pharmaceutique selon la revendication 10, comprenant en outre un mélange avec des excipients sélectionnés parmi le groupe comprenant des agents de charge, des correcteurs de goût, des parfums, et des substances odoriférantes.
